# EUROPÄISCHE PATENTSCHRIFT

(11) **EP 2 032 970 B1**
(45) Veröffentlichungstag und Bekanntmachung des Hinweises auf die Patenterteilung: **19.12.2012**
(21) Anmeldenummer: 07722533.2
(22) Anmeldetag: 17.06.2007
(51) Int. Cl.: G01N 21/64, A61B 5/00, G01J 3/44

(54) **ORTSAUFGELÖSTES MESSVERFAHREN FÜR DIE DETEKTION VON MELANIN IN FLUOROPHORGEMISCHEN IN EINER FESTKÖRPERPROBE**
SPATIALLY-RESOLVED MEASUREMENT METHOD FOR THE DETECTION OF MELANIN IN FLUOROPHOR MIXTURES IN A SOLID SAMPLE
PROCÉDÉ DE MESURE LOCALE POUR LA DÉTECTION DE MÉLANINE DANS DES MÉLANGES DE FLUOROPHORE DANS UN PRÉLÈVEMENT DE SOLIDE

(30) Priorität: 28.06.2006 DE 102006029809
(43) Veröffentlichungstag der Anmeldung: 11.03.2009
(73) Patentinhaber: LTB-Lasertechnik Berlin GmbH, 12489 Berlin (DE)
(72) Erfinder: SCHOLZ, Matthias, 15838 Am Mellensee (DE); LEUPOLD, Dieter, 14612 Falkensee (DE)
(86) Internationale Anmeldenummer: PCT/DE2007/001076
(87) Internationale Veröffentlichungsnummer: WO 2008/000223

(56) Entgegenhaltungen:
- DE-A1- 10 239 028
- US-A- 5 555 885
- US-A1- 2004 073 119
- ANONYM: "Selektive Melaninfluoreszenz informiert über Hautkrebs" NEWS LTB LASERTECHNIK BERLIN, [Online] 21. Juli 2006 (2006-07-21), XP002450034 Gefunden im Internet: URL:http://www.ltb-berlin.de/de_info_20.ht ml> [gefunden am 2007-09-06]
- TEUCHNER KLAUS ET AL: "Femtosecond two-photon excited fluorescence of melanin" PHOTOCHEMISTRY AND PHOTOBIOLOGY, Bd. 70, Nr. 2, August 1999 (1999-08), Seiten 146-151, XP009089146 ISSN: 0031-8655 in der Anmeldung erwähnt
- TEUCHNER K ET AL: "Fluorescence studies of melanin by stepwise two-photon femtosecond laser excitation" JOURNAL OF FLUORESCENCE KLUWER ACADEMIC/PLENUM PUBLISHERS USA, Bd. 10, Nr. 3, September 2000 (2000-09), Seiten 275-281, XP002450035 ISSN: 1053-0509 in der Anmeldung erwähnt

## Beschreibung

Die Erfindung bezieht sich auf ein ortsaufgelöstes Verfahren zur Detektion von Melanin in Fluorophorgemischen in einer Festkörperprobe durch Fluoreszenzanregung ausschließlich des im Fluorophorgemisch vorhandenen Melanins durch Photonen-Absorption mit zumindest einem Puls einer Laserlichtquelle und Erfassung des im Fluorophorgemisch vorhandenen Melanins aus dessen ausgesendeter spektraler Fluoreszenzantwort durch Auswertung der Anzahlen der ausgesendeten Photonen.

Fluoreszenzuntersuchungen zur Identifizierung bestimmter Stoffe sind seit langem bekannt. Die Fähigkeit, nach Photonenabsorption Licht zu emittieren, d.h. zu lumineszieren, ist stoffspezifisch. Dies ist die Grundlage der konventionellen Lumineszenzanalytik. Es sind heute mehrere Millionen lumineszierender, d.h. fluoreszierender und/oder phosphoreszierender organischer Verbindungen bekannt, und häufig kommen in einem zu untersuchenden Material mehrere lumineszierende Stoffe vor. Dies gilt z.B. häufig für Meßproben/Fragestellungen aus den Biowissenschaften und der Medizin. So enthält beispielsweise menschliches Hautgewebe wenigstens zehn verschiedene endogene Fluorophore, dazu kommen weiterhin exogene Fluorophore, und daher ist das Autofluoreszenzspektrum der Haut die Resultierende aus vielen individuellen Fluoreszenzbanden. Es sind verschiedene, in der Regel in Kombination anzuwendende Verfahren bekannt, um bei Fluorophorgemischen Zugang zu einer Komponentenanalyse zu bekommen, z.B durch Variation der Anregungswellenlänge, durch Anregungsspektren in Abhängigkeit von den Fluoreszenzwellenlängen, Fluoreszenz-Abklingverhalten und Polarisationsspektren, aber die Anwendung der kombinierten Methodik ist nicht nur zeitaufwendig, sondern kann z.B. in Fällen, bei denen das Fluorophorgemisch in einer Matrix vorliegt, durch deren eigene optische Eigenschaften, wie Eigenabsorption und Streuung, nur begrenzt anwendbar sein. Eine weitere Komplizierung der Analyse von Fluorophorgemischen in Matrizen tritt auf, wenn sie hinsichtlich ihrer optischen Eigenschaften in sich inhomogen sind und wenn die Zusammensetzung des Fluorophorgemisches in diesen inhomogenen Matrizen noch zusätzlich selbst eine Funktion des Ortes ist. Eine solche Situation liegt bei der Matrix menschlichen Hautgewebes mit dem dort vorkommenden Gemisch endogener und exogener Fluoröphore vor. Die Fluorophor-Komponentenanalyse wird bei dieser Matrix dadurch zusätzlich erschwert, dass sie für sichtbares Licht eine vom langwelligen zum kurzwelligen stark abnehmende Eindringtiefe aufweist. Diesem Nachteil kann durch nichtlineare Fluorophor-Anregung mittels simultaner Zweiphotonenabsorption im langwelligen Spektralbereich begegnet werden, aber dies schränkt nicht nur die o.g. breite Methodenkombination für die Fluorophor-Komponentenanalyse erheblich ein, bzw. macht sie außerordentlich aufwendig, sondern bedingt vor allem auch den Einsatz ultrakurzer intensiver, hochrepetierender Laser-Lichtpulse im Femtosekundenbereich (fs). Damit ist die weitgehend bekannte Gefahr eines photochemischen Ausbleichens der Fluorophore verbunden und insbesondere bei in-vivo Anwendungen besteht außerdem das Risiko der Beeinflussung der Zellteilungsrate, ausgelöst durch die erforderlichen hohen Photonenflußdichten von typischerweise ≥10²⁹ Photonen pro cm² und s und hochrepetierender Bestrahlungsregimes.

Nun ist aber gerade die Fluorophor-Komponentenanalyse in menschlichem Gewebe von erheblichem Interesse, z.B. im Zusammenhang mit medizinischdiagnostischen, pharmazeutischen und kosmetischen Fragestellungen. Dabei steht ganz besonders der endogene Fluorophor Melanin im Vordergrund. Melanin kommt u.a. in der Haut, in Haaren und im Auge vor, es ist z.B. verantwortlich für die Haut- und Haarfarbe, und es spielt insbesondere eine zentrale Rolle einerseits als "Sonnenschirm", andererseits bei der Entartung von Hautgewebe zum malignen Melanom, dem schwarzen Hautkrebs. Nach S. P. Nighswander-Rempel et. al. "A quantum yield map for synthetic eumelanin" in J. Chem. Phys. 123, 2005, 194901-1-6 hat Melanin den für eine Fluoreszenzanalytik gravierenden Nachteil einer außerordentlich geringen Fluoreszenzquantenausbeute in der Größenordnung von höchstens 10⁻⁴, eine aus der ungewöhnlichen Absorption von Melanin abgeleitete spezifische Fluoreszenzquantenausbeute liegt sogar nur in der Größenordnung von 10⁻⁶. Das Absorptionsspektrum des Melanins ist verschieden von dem nahezu sämtlicher anderer organischer Fluorophore. Während letztere zwischen nahem Ultraviolett- und nahem Infrarot-Spektralbereich nur einzelne diskrete Absorptionsbanden zeigen, hat Melanin im genannten Spektralbereich einen monoton fallenden Absorptionsverlauf. Somit gibt es bei Melanin enthaltenden Fluorophorgemischen im Falle der Zweiphotonenabsorption im roten bzw. nahen infraroten Spektralbereich spektral keine auch nur annähernd selektive Anregung des Melanins, denn jede Lichtwellenlänge, die irgendeinen Fluorophor anregt, regt auch Melanin an. Aus der DE 199 39 706 C2 ist es zwar bekannt, dass sich durch Zweiphotonen-Anregung mit Femtosekunden-Impulsen eine Anreicherung des angeregten Melanins gegenüber allen anderen Fluorophoren erreichen lässt, womit quasi eine gewisse Kompensation der geringen Fluoreszenzquantenausbeute möglich ist. Dies basiert darauf, dass die Zweiphotonen-Anregung beim Melanin als stufenweiser Prozeß zweier aufeinanderfolgender Einphotonen-Absorptionen über ein reelles.Zwischenniveau verläuft (siehe K. Teuchner et. al. "Femtosecond Two-photon Excited Fluorescence of Melanin" in Photochem. Photobiol. 70(2), 1999, pp.146-151), im Unterschied zur üblichen simultanen Zweiphotonen-Anregung mit einem nur virtuellen Zwischenniveau bei den relevanten anderen Fluorophoren. Aber die fluoreszenzspektroskopische Signifikanz und analytische Verwertbarkeit dieser Anreicherung von angeregtem Melanin ist begrenzt durch dessen im Vergleich zu den anderen relevanten Fluorophoren außerordentlich geringe Fluoreszenzquantenausbeute. Aus der Veröffentlichung von K. Hoffmann et. al. "Selective Femtosecond Pulse-Excitation of Melanin Fluorescence in Tissue" J. Invest. Dermatol. 116 (2001), 629-630, ist zwar bekannt, dass mit dieser auf Femtosekundenpulsen basierenden Zweiphotonen-Anregung an malignen Melanomen ex-vivo eine Rotverschiebung der Fluoreszenz gegenüber gesundem Hautgewebe ex-vivo gemessen werden kann und eine Verkürzung des Fluoreszenzabklingens auftritt (siehe auch DE 102 39 028 B4). Zu den oben erwähnten lokalen Ausbleicheffekten mit noch unbekannten Folgereaktionen und der Gefahr der Beeinflussung der Zellteilungsrate bei Pulsleistungsdichten ab 100 GW/cm² kommt bei der nichtlinearen Fluorophor-Anregung in-vivo mit intensiven, ultrakurzen Lichtpulsen eine Gefahrenquelle hinzu, die bislang praktisch völlig unbeachtet geblieben ist: Die gefährlich hohe Effektivität einer unerwünschten Dreiphotonen-Anregung von Fluorophoren, die aus einer der simultanen Zweiphotonenabsorption nachfolgenden Einphotonenabsorption aus dem Anregungszustand resultiert. In der Literatur ist eine bei der nichtlinearen Fluorophoranregung mögliche Dreiphotonenabsorption bislang nur selten erwähnt und dann im Sinne einer simultanen Dreiphotonenabsorption insofern falsch interpretiert worden, als dass sie infolge ihres außerordentlich geringen Wirkungsquerschnitts eine verschwindend geringe Effektivität aufwiese. Mit dieser Fehlinterpretation wird die konkrete Gefahr nicht erkannt, die von einer tatsächlich auftretenden Zweiphotonenabsorption mit nachfolgender Einphotonenabsorption ausgeht. Dieser Prozess entspricht energetisch einer Anregung im UV-B bzw. sogar UV-C Bereich. Deren hohes karzinogenes Potential macht die Anwendung der Methode der nichtlinearen Fluorophor-Anregung mit Femtosekundenimpulsen auf menschliches Gewebe sehr risikoreich und erhöht z.B. die Gefahr von DNA-Protein Cross-links. Die beschriebenen Effekte lassen diese Form der Melanom-Diagnostik auf der Basis von Femtosekunden-Impulsen für in-vivo Anwendungen als zu risikoreich erscheinen.

Aus der US 5,034,613 ist ein Laser-Mikroskop mit simultaner Zweiphotonen-Fluoreszenz-Anregung bekannt, das zur Untersuchung von Zellmaterial Anregungswellenlängen im Bereich von rotem zu nahem infrorotem Licht, d.h. zwischen 640 und 1200 nm, mit Pulslängen im Subpicosekundenbereich, d.h. <10⁻¹² s, hier mit 100 Femtosekungen (fs), mit einer Wiederholrate von 80 MHz verwendet. Es kommt eine sehr hohe lokale Lichtintensität durch eine Fokussierung auf 1µm zustande. Durch die sehr enge Fokussierung soll das Ausbleichen der Fluorophore auf den unmittelbaren Beobachtungsbereich begrenzt werden. Ferner soll die Zweiphotonen-Anregung die sogenannte Hintergrundfluoreszenz verstärkt unterdrücken. Aus der DE 44 14 940 C2 ist ein Lumineszenz-Rastermikroskop mit Zweiphotonen-Anregung bekannt, das mit Laserpulsen größer als 1 Picosekunde (ps) arbeitet, um die Verwendung teurer Femtosekunden-Laser zu vermeiden. Um die dabei zur Schonung der Untersuchungsobjekte eingesetzte niedrige Pulsleistung auszugleichen, wird eine größere Messdauer, d.h. eine längere Pulsfolge zur Lumineszenzanregung eingesetzt. Aus der DE 197 19 344 A1 ist eine Anordnung zur optischen Mikromanipulation, Analyse, und Beabeitung von Objekten bekannt, die mit einem Wellenlängenspektrum zur Anregung im Bereich zwischen 400 und 1200 nm und Pulslängen in Nano-, Pico- und Femtosekundenbereich arbeitet. Die Anordnung bezieht sich im Wesentlichen auf den Einsatz eines über den gesamten Spektralbereich durchstimmbaren Lasers und weniger auf die zur eigentlichen Stoffanalyse bestimmte Fluoreszenzanregung. Dennoch wird ausdrücklich darauf hingewiesen, dass zur Analyse nur die Pulsdauern im Femtosekundenbereich eingesetzt werden. Pulslängen im Bereich von Picosekunden oder länger werden ausschließlich für die Mikromanipulation verwendet.

Aus der DE 199 35 766 A1 ist ein Verfahren zur optischen Anregung von Fluorophor markierter DNA und RNA bekannt, bei dem eine simultane, nicht resonante Multiphotonen-Fluoreszenzanregung mit bevorzugten Wellenlängen aus dem Bereich zwischen 760 und 820 nm und Leistungsdichten zwischen 100 MW/cm² und 10 TW/cm² eingesetzt wird. Es wird herausgestellt, dass die simultane Zweiphotonen- oder Dreiphotonen-Anregung in der hier angesprochenen DNAIRNA-Analytik bisher nicht bekannt ist. Insbesondere wird ein Beispiel herausgestellt, bei dem verschiedene Fluorophore mit einer Wellenlänge von 770 nm, einer Pulsdauer von 200 fs, ein Pulsfrequenz von 76 MHz und einer Leistungsdichte von 500 GW/cm² zu einem kontrastreichen Fluoreszenzspektrum mit Maxima zwischen 480 und 650 nm angeregt werden konnten. Die DE 199 39 706 C2 beschreibt die Auswahl von Fluorophoren für die Stoffmarkierung bei der Multiphotonen-Laserscanning-Mikroskopie, die eine stufenweise, resonante Absorption mit reellen Zwischenniveaus aufweisen. Dabei soll zur Anregung eine wesentlich geringere Laserintensität, d.h. Photonenflussdichte, erforderlich sein, so dass einerseits der gerätetechnische Aufwand verringert und andererseits die Gefahr elektrischer Durchschläge und der photochemische Effekt des Ausbleichens der Stoffprobe nach der Einphotonen-Absorption minimiert werden kann. Insbesondere wird künstliches Melanin als ein solches Fluorophor hervorgehoben, bei dem der Wirkungsmechanismus der stufenweisen, resonanten Multiphotonen-Absorption gezielt ausgenutzt, d.h. die Anregung nicht über virtuelle sondern über reelle Zwischenniveaus erzielt wird. Konkret wird eine Wellenlänge von 800 nm, eine Pulsdauer von 120 fs und eine Pulsenergie von 1 µJ zur Fluoreszenz-Anregung verwendet. Die emittierte Fluoreszenz liegt im blaugrün-roten Spektralbereich mit einem Maximum bei 610 nm. Die DE 100 65 146 A1 beschreibt ein Verfahren und eine Anordnung zur nicht invasiven dreidimensionalen optischen Untersuchung und Therapie der Haut, die gepulste Laserstrahlung im nahen Infrarotbereich mit Wellenlängen von 700 bis 1200 nm sowie Pulslängen von weniger als 20 ps mit Lichtintensitäten in der Größenordnung zwischen Gigawatt pro cm² und Terrawatt pro cm² bei einer Pulsfolgefrequenz von 80 MHz zur Multiphotonen-Anregung körpereigener Fluorophore verwenden. Insbesondere sollen Melanome der Haut lokalisiert und irreversibel geschädigt werden. Es wird beschrieben, dass resonante und nicht resonante Multiphotonen-Fluoreszenzanregung spezifischer endogener Fluorophore, insbesondere Melanin, hervorgerufen wird, wodurch anhand der festgestellten Anordnung der Fluoreszenz-Intensität und der Fluoreszenz-Lebensdauer eine Unterscheidung nach bestimmtem pathologischem und gesundem Gewebe möglich sein soll. Auf die exakten Wirkmechanismen der Multiphotonen-Anregung im Zusammenhang mit den Anregungsparametern sowie die Interpretation der Fluoreszenzantwort zur exakten Lokalisierung pathologischen Gewebes wird nicht eingegangen.

In der WO 02/069784 wird ein tragbares Fluoreszenz-Lebenszeit Spektrometer (FLS) zur gleichzeitigen in-vivo-Analyse der spektralen und temporalen Fluoreszenzeigenschaften von Gewebe oder Zellen auf karzinogene oder prekarzinogene Gewebeanteile beschrieben. Die zeitabhängige Fluoreszenzantwort endogener Fluorophore wie Kollagen, Elastin, NADPH und Tryptophan ist stark von der biochemischen Umgebung und deren pH-Wert und Sauerstoffgehalt abhängig, wodurch auf gesundes oder krankes Gewebe geschlossen werden kann. Das FLS kann in weniger als einer Sekunde die Daten des transienten Abklingverhaltens eines bestimmten Frequenzbandes der Fluoreszenz des untersuchten Gewebes über Zeiträume von durchschnittlich 360 Picosekunden verarbeiten und ist damit für den in-vivo-Einsatz geeignet. Die Druckschrift stellt damit kein neues Messverfahren, sondern ein für einen speziellen Zweck optimiertes Messgerät vor.

Die DE 102 39 028 B4, von der die vorliegende Erfindung als nächstliegendem Stand der Technik ausgeht, beschreibt ein Verfahren zur Identifizierung von natürlich vorkommenden oder synthetisch hergestellten Melaninsorten. Dabei wird das vorkommende Melanin durch Einphotonen- und stufenweise, resonante Zweiphotonen-Anregung selektiv gegenüber anderen in der Probe vorhandenen Fluorophoren mit Laserpulsen der Wellenlänge 800 nm und einer Pulslänge im Femtosekundenbereich angeregt und das als Antwort darauf erhaltene Fluoreszenzspektrum nach spektraler Verteilung und zeitlich aufgelöst ausgewertet. Durch die spektrale Verteilung der erhaltenen Fluoreszenz-Intensitäten und das Abklingverhalten kann nach den verschiedenen Melaninsorten selektiv unterschieden und damit eine Aussage über das Vorhandensein von Gewebe mit dem Verdacht auf maligne Melanome entschieden werden.

Im Stand der Technik wird die Detektion von Fluorophoren im Allgemeinen und Melanin im Besonderen regelmäßig mit Laserpulsen mit Pulslängen im Femtosekundenbereich, höchstens jedoch mit kleiner 20 ps angeregt. Der Wellenlängenbereich wird mit 700 bis 1200 nm angegeben, wobei regelmäßig eine Wellenlänge von 800 nm zur Anwendung kommt. Die energiereichen Pulse werden hoch repetitiv mit Frequenzen von z.B. 80 MHz abgestrahlt und erzeugen Leistungsdichten des Photonenflusses zwischen 100 GW/cm² und einigen TW/cm². Mit den Pulslängen im Femtosekundenbereich ist hoher apparativer Aufwand verbunden, der den Einsatz handlicher und einfach bedienbarer Geräte, z.B. für die Melanomdiagnostik, unmöglich macht. Wenn die weiter oben beschriebenen Nachteile und Risiken zu Gunsten der Möglichkeit der selektiven Melanin-Detektion nicht in Kauf genommen werden sollen, müssen Diagnostikverfahren mit der Absicht einer in-vivo Anwendung mit Leistungsdichten deutlich unter 100 GW/cm², der Schwelle für Zellschädigungen, arbeiten. Aus den neuesten Erkenntnissen zur Gefahr einer hinter einer vermeintlichen Zweiphotonenabsorption verdeckt auftretenden tatsächlichen Dreiphotonenabsorption mit karzinogenem UV-C Potential resultiert eine deutlich verschärfte Bedingung von Leistungsdichten ≤ 1 GW/cm² . Insbesondere muß der hohe Energieeintrag in die Gewebe-Matrix, verursacht durch die auf hochrepetierenden Lasersystemen basierenden Meßsysteme, strikt vermieden werden, wenn die zu untersuchenden Fluorophorgemische Melanin enthalten, da praktisch die gesamte im Melanin absorbierte Energie im Gewebe verbleibt und z.B. in Wärme oder in photochemischen Folgeprozessen umgesetzt werden muß. Dies ist ein Spezifikum der außerordentlich geringen Fluoreszenzquantenausbeute von Melanin. Andere Fluorophore geben typischerweise einen Großteil der absorbierten Anregungsenergie wieder als Fluoreszenz-Strahlung ab.

### Aufgabenstellung

Die **Aufgabe** für die vorliegende Erfindung ist daher darin zu sehen, ein gattungsgemäßes ortsaufgelöstes Detektionsverfahren für Melanin in Fluorophorgemischen in einer Festkörperprobe der eingangs genannten Art so weiterzubilden, dass die Nachteile durch hohen apparativen Aufwand, aufwendige Handhabung, mehrstufige Verfahren und nicht eindeutige Detektionsergebnisse und insbesondere wegen hoher Einstrahlleistungen bestehende Risiken durch Ausbleichen, Beeinflussung von Zellteilungsmechanismen, Verbrennungen und Krebsgefahr bei in-vivo-Detektionen vermieden werden und ein einfaches, eindeutige Ergebnisse lieferndes, handhabbares und preisgünstiges Verfahren zur Verfügung gestellt wird. Die **Lösung** für diese Aufgaben ist dem Hauptanspruch zu entnehmen. Vorteilhafte Modifikationen werden jeweils in den Unteransprüchen aufgezeigt und im Folgenden im Zusammenhang mit der Erfindung näher erläutert.

Das erfindungsgemäße ortsaufgelöste Detektionsverfahren für Melanin in Fluorophorgemischen in einer Festkörperprobe erzielt seine aufgabengemäße Wirkung durch ausschließliche Fluoreszenz-Anregung des im Fluorophorgemisch vorhandenen Melanins durch Photonen-Absorption mit zumindest einem Puls einer Laserlichtquelle, die mit einer Pulslänge von 0,5 bis 5 ns eine Anregungswellenlänge im Bereich zwischen 300 und 1000 nm mit einer Photonenflussdichte von 10²⁶ bis 10²⁸ Photonen pro cm² und s abstrahlt. Die Detektion erfolgt durch Zählung der im Fluoreszenzspektrum zwischen 400 und 700 nm abgestrahlten Photonen.

Es hat sich nämlich besonders überraschenderweise gezeigt, dass im Gegensatz zum bisherigen Kenntnisstand in der Literatur (K. Teuchner et al. J. Fluor. 10/3, 2000, 275-281 J. Fluor. 10/3, pp. 275-281, 2000) das durch Zweiphotonen-Anregung hervorgerufene Fluoreszenzspektrum des Melanins auch mit Laserpulsen mit Pulslängen größer als um 100 fs herum angeregt werden kann, insbesondere mit technisch bequem realisierbaren Pulsen im Nanoskundenbereich, bei einer besonder vorteilhaften Weiterbildung des Verfahrens nach der Erfindung mit einer Pulsdauer von 2 ns. Dies ist für die vorliegende Aufgabenstellung in mehrerer Hinsicht von herausragender Bedeutung, wie sich schon theoretisch und anschaulich an der Abhängigkeit der Besetzungsdichte des mittels Zweiphotonenabsorption besetzten Fluoreszenzniveaus von der Impulsdauer (siehe DE 199 39 706 C2) verdeutlichen lässt: Eine Verlängerung der Impulsdauer um 4 Größenordnungen (100fs → 1 ns) bewirkt bei sonst unveränderten Parametern bei der für übliche Fluorophore simultanen Zweiphotonen-Absorption eine Erhöhung der Besetzungsdichte im Fluoreszenzniveau um gleichfalls 4 Größenordnungen, für stufenweise Zweiphotonen-Absorption bei Melanin hingegen um 8 Größenordnungen. Da für beide Typen von Zweistufen-Absorption diese Besetzungsdichte quadratisch von der Anregungsintensität abhängt, könnte die Laser-Pumpintensität der Pulse im Nanosekundenbereich theoretisch um 4 Größenordnungen gegenüber den Pulsen um 100 fs herum vermindert werden, um für Melanin bei Anregung im Nanosekundenbereich dieselbe Fluoreszenzintensität zu erhalten wie im Verfahren mit Impulsen um 100 fs herum. Die Fluoreszenz der anderen, üblichen Fluorophore hätte sich demgegenüber um 4 Größenordnungen vermindert, d.h. sie wäre vergleichsweise unmessbar. Dies bestätigt sich angenähert so auch in der Praxis. Um bei gleichem Detektionssystem bei Anregung im Nanosekundenbereich eine deutliche selektive Fluoreszenzantwort des Melanin zu erhalten, kann die Photonenflußdichte um nahezu 3 Größenordnungen gegenüber Anregung um 100 fs herum vermindert werden und nach einer vorteilhaften Weiterbildung des Verfahrens nach der Erfindung bei 10²⁷ Photonen pro cm² und s, entsprechend etwa 300 MW/cm² für Photonen des roten bis NIR Spektralbereichs, liegen. Die anderen üblichen Fluorophore sind bei dieser Anregung und unter identischen Detektionsbedingungen unmessbar schwach, d.h. eine sichere selektive Melanin-Fluoreszenzdetektion aus dem vorliegenden Fluorophorgemisch ist realisiert. Dabei sind gleichzeitig die erforderlichen Laserintensitäten um mehrere Größenordnungen vermindert worden, d.h. die Gefahr der o.g. Strahlenschädigungen ist drastisch reduziert. Zur Detektion der Melanin-Fluoreszenz kann nach weiteren vorteilhaften Ausbildungen des Verfahrens nach der Erfindung eine Mittelung über eine Akkumulationszahl von 2 bis 100 einzelner Pulsen der Laserlichtquelle bei vorteilhaft geringem Energieeintrag durchgeführt und die spektrale Fluoreszenzantwort in Wellenlängenschritten von 25 nm erfasst werden.

Nach weiteren vorteilhaften Ausbildungen des Verfahrens nach der Erfindung kann die Fluoreszenzanregung durch Anregungswellenlängen im Bereich zwischen 300 und 350 nm ausschließlich durch Einphotonen-Absorption und die Erfassung der Fluoreszenzantwort global über die Festkörperprobe durch eine bildliche Darstellung erfolgen. Dabei kann besonders vorteilhaft die Anregungswellenlänge 337 nm betragen und die bildliche Darstellung durch direkte optische Fotografie der im sichtbaren Bereich abgestrahlten Fluoreszenz erfolgen. Es hat sich nämlich überraschend gezeigt, dass Hautgewebeareale mit malignen Melanomen eine charakteristische Strukturierung in der Intensitätsverteilung des Fluoreszenzbildes des Gesamtareals aufweisen, wenn dieses mit Einphotonen-Anregung erzeugt und mit einem hochempfindlichen, gegateten Detektionssystem aufgenommen wird. Geeignet sind dazu besonders Anregungen mit einem Nanosekundenimpuls-Stickstofflaser (337 nm) und spektral selektierte Fluoreszenz im Bereich 400-650 nm. Es hat sich außerdem überraschend herausgestellt, dass in malignen Melanomen eine solche Verarmung an Fluoreszenz der üblicherweise bei konventioneller Einphotonenanregung dominierenden Fluorophore auftritt, dass bei gemäß den vorteilhaften Ausprägungen des Verfahrens gewählten Anregungs- und Detektionswellenlängen diese Fluoreszenzlöschung als ein erstes Indiz für maligne Entartung benutzt werden kann. Daher kann die ortsaufgelöste Detektion interessierender Hautgewebsareale mit dem Verfahren nach der Erfindung im Fluoreszenzlicht von mit geeigneten UV-Photonen aus Nanosekundenimpulsen angeregten Fluorophoren, spektral gefiltert, zur Erkennung von Verdachtsarealen für maligne Melanome eingesetzt werden.

Nach weiteren vorteilhaften Ausbildungen des Verfahrens nach der Erfindung kann die Fluoreszenzanregung durch Anregungswellenlängen im Bereich zwischen 600 und 1000 nm ausschließlich durch stufenweise Zweiphotonen-Absorption und die Erfassung der Fluoreszenzantwort lokal und selektiv bei der für die Melaninsorte *Eumelanin* charakteristischen Fluoreszenzwellenlänge von 475 nm und bei der für die Melaninsorte *Pheomelanin* charakteristischen Fluoreszenzwellenlänge von 575 nm erfolgen. Dabei kann besonders vorteilhaft die Anregungswellenlänge zwischen 800 und 900 nm liegen und die lokale Ortsauflösung bei Messspots im Bereich zwischen 40 und 100 µm liegen. Zur weiteren Risiko-Minimierung bei in-vivo Fluoreszenzmessungen kann die Anregungswellenlänge von den sonst standardmäßig verwendeten 800 nm auf Wellenlängen um 900 nm herum verschoben werden. Damit wird schon durch die vorgegebene Laser-Wellenlänge ein Erreichen des kritischen UV-Bereichs unterhalb von 300 nm bei nichtlinearer Dreiphotonen-Absorption ausgeschlossen. Die Energie von 3 hv für λ = c/v =900 nm entspricht einer Wellenlänge von 300 nm. Diese energetisch wesentliche Verschiebung der Anregungswellenlänge für Melanin auf den Bereich um 900 nm herum wird hier erstmals beschrieben und erfindungsgemäß eingesetzt. Es hat sich nämlich weiterhin besonders überraschend gezeigt, dass Melanin auch noch mit Impulsen mit einer Wellenlänge um 900 nm herum zu einer einwandfrei messbaren, stufenweise angeregten Fluoreszenz angeregt werden kann. Eine solche Melaninfluoreszenz, z.B. im menschlichen Hautgewebe, zeigt ex-vivo im Paraffinschnitt ein spektral breites, asymmetrisches Profil mit einem Maximum im blaugrünen Spektralbereich bei etwa 475 nm und einer lang auslaufenden Flanke bis in den roten Spektralbereich, die implizit eine zweite Komponente darstellt. Es hat sich dabei außerdem überraschend gezeigt, dass sich im Falle der Entartung des Hautgewebes zum malignen Melanom das Spektralprofil der Melaninfluoreszenz signifikant verändert. Es zeigt nun deutlich zwei Banden, im Paraffinschnitt 475 nm und 575 nm, d.h. der gelbrote Spektralanteil der Fluoreszenz ist wesentlich deutlicher ausgeprägt. Dieser Umstand folgt aus der signifikanten Abnahme der Gesamtfluoreszenz des Melanins im malignen Melanom gegenüber gesundem Gewebe, und diese Abnahme geht zu Lasten der kurzwelligen Komponente. Diese erstmals durch die Wahl der erfindungsgemäßen Verfahrensparameter erhaltenen reinen Fluoreszenzspektren von Melanin bei Anregung des Fluorophorgemisches in Hautgewebe machen deutlich, dass alle früher ausschließlich mit Impulsen im Femtosekundenbereich gemessenen Fluoreszenzspektren von Hautgewebe keine reine Melaninfluoreszenz widerspiegeln, sondern auch Anteile von Fluorophoren mit simultaner Zweiphotonen-Absorption. Das zeigt sich explizit bei der Fluoreszenzdetektion von Hautgewebe mit malignem Melanom in Paraffin, bei dem ein und dasselbe Gewebeareal sowohl mit 800 nm Impulsen im Femtosekundenbereich als auch mit Impulsen im Nanosekundenbereich angeregt wurde. Das dabei auftretende Minimum bei 525 nm bei der Fluoreszenzanregung im Nanosekundenbereich, ist bei der Fluoreszenzanregung im Femtosekundenbereich durch die FAD-Fluoreszenz (Flavin-Adenin Dinukleotid) überdeckt. Insofern zeichnet sich also das erfindungsgemäße Verfahren eindeutig durch eine größere Nachweisempfindlichkeit hinsichtlich durch krankhafte Veränderungen bedingter Melaninfluoreszenz in Hautgewebe aus. Diese durch das erfindungsgemäße Verfahren gesteigerte Nachweisempfindlichkeit kommt insbesondere der Erkennung beginnender maligner Entartung in Naevi zugute und ist für maligne Entartungen ein weiterer wesentlicher Vorteil neben dem Vorteil der Nichtinvasivität, d.h. Eliminierung der verschiedenen oben dargestellten Gefahren der Strahlenbelastung und dem Vorteil des deutlich geringeren apparativen Aufwandes für Impulse im Nanosekundenbereich im Vergleich zu Impulsen im Femtosekundenbereich.

In einer weiteren vorteilhaften Ausführungsform des Verfahrens nach der Erfindung werden beide bisher beschriebenen Verfahrensausprägungen zusammengefasst. Dazu wird die Fluoreszenz-Detektion global zu untersuchender Hautareale im Licht einer durch Einphotonen-Absorption angeregten Fluoreszenz durchgeführt und die Auswahl von Probenarealen speziellen Interesses durch Feststellung von Fluoreszenzauslöschungen in der Fluoreszenzantwort getroffen. Anschließend wird in diesen ausgewählten Arealen eine mittels der stufenweisen Zweiphotonen-Absorption angeregte Fluoreszenzantwort lokal und selektiv erfasst und der auftretende Anteil insbesondere der Melaninsorte *Pheomelanin* durch Feststellung der zugehörigen Anzahl der ausgestrahlen Photonen ermittelt. Der Grad der im ersten Verfahrensabschnitt zu ermittelnden Fluoreszenzauslöschung kann dabei vorteilhaft durch proportional dazu in der büdhaften Darstellung auftretende Farb- oder Grauabstufungen festgestellt werden, wobei die dunkelsten Areale mit der größten Fluoreszenzauslöschung für die weitere Untersuchung im zweiten Verfahrensabschnitt ausgewählt werden. Vorteilhaft kann auch das Verhältnis der auftretenden Anteile der Melaninsorten *Eumelanin* und *Pheomelanin* registriert werden. Weiterhin kann das Verfahren besonders vorteilhaft auf Festkörperproben von Gewebeteilen der menschlichen Haut, des menschlichen Augenhintergrunds oder menschlicher Haare angewendet werden, wobei das Verfahren entweder ex-vivo an in Parafin stabilisierten und Formalin fixierten oder in-vivo an in ihrer natürlichen Umgebung fixierten Festkörperproben durchgeführt werden kann. Insgesamt kann das ortsaufgelöstes Messverfahren für die Detektion von Melanin in Fluorophorgemischen in einer Festkörperprobe nach der Erfindung besonders vorteilhaft für die technisch objektive Früherkennung von malignen Melanomen in menschlichem Hautgewebe als Festkörperprobe eingesetzt werden.

### Ausführungsbeispiele

Das ortsaufgelöste Messverfahren für die Detektion von Melanin in Fluorophorgemischen in einer Festkörperprobe nach der Erfindung wird nachfolgend anhand der schematischen Figuren beispielhaft näher erläutert. Dabei zeigt:
- **Figur 1A**: Fluoreszenzspektren eines Melanoms, aufgenommen mit Anregungspulsen bei 810 nm und 2 ns; mit Fotos der Messorte,
- **Figur 1B**: Fluoreszenzspektren eines Melanoms, aufgenommen mit Anregungspulsen bei 880 nm und 2 ns; mit Fotos der Messorte,
- **Figur 2**: Vergleich zweier Fluoreszenzspektren eines Melanoms und gesunder Haut, aufgenommen mit Anregungspulsen bei 810 nm und 0,7 ns,
- **Figur 3A**: Foto eines Hautareals mit malignem Melanom
- **Figur 3B**: Grauwert-Übersichtsfoto des unter 3A genannten Hautareals im Lichte der mit 337 nm -Anregungspulsen erzeugten Fluoreszenz
- **Figur 3C**: Fluoreszenzspektren an den im Foto Figur 3B angegebenen Messorten mit 810 nm und 2 ns Zweiphotonen-Anregungspulsen
- **Figur 4A**: Anordnung zu Aufnahmen im Licht der Fluoreszenz bei Einphotonen-Anregung
- **Figur 4B**: Messanordnung für Fluoreszenzspektren bei Zweiphotonen-Anregung

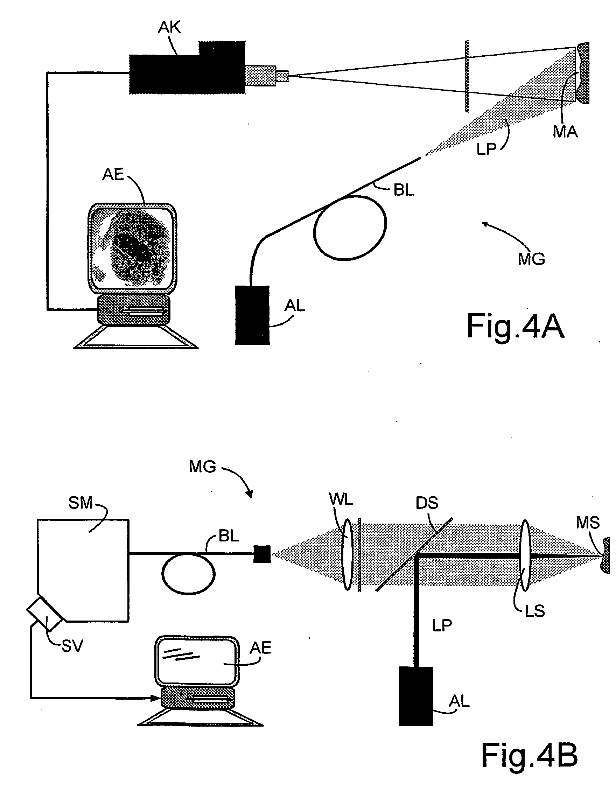

Alle in den nachfolgend beschriebenen Figuren angegebenen Zahlenwerte und Abbildungen zu Fluoreszenzspektren beziehen sich auf unter vergleichbaren Versuchsbedingungen mit den in Figur 4 dargestellten Messanordnungen ermittelte, spektral nicht korrigierte Fluoreszenzspektren.

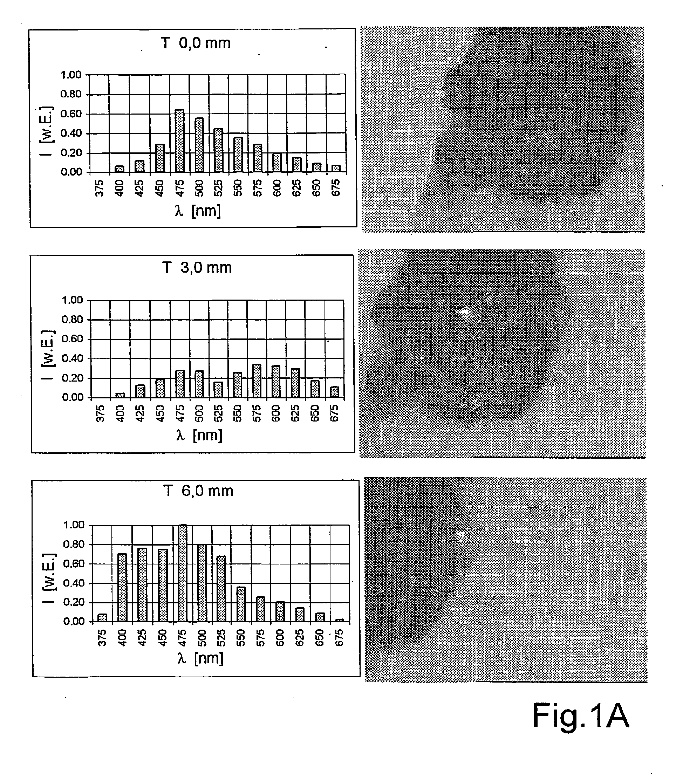

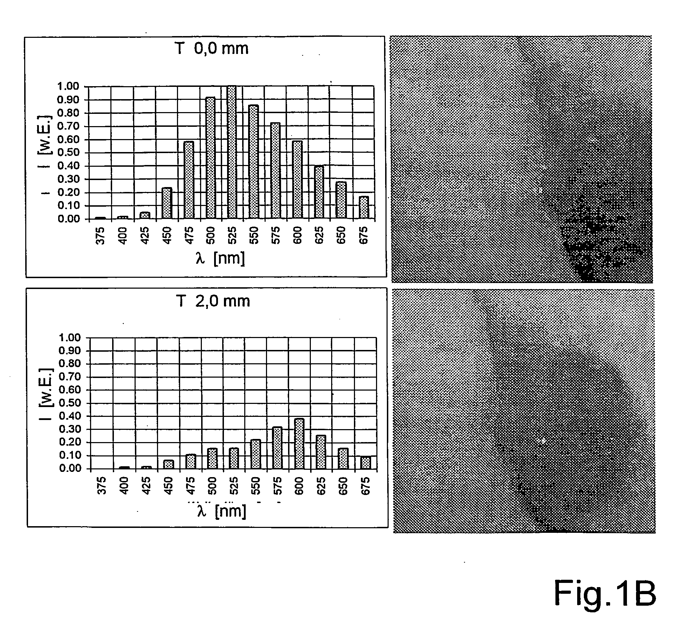

In der **Figur 1A** sind drei Fluoreszenzspektren eines malignen Melanoms mit Zweiphotonen-Anregung und Anregungspulsen bei 810 nm Wellenlänge und 2 ns Pulslänge mit Fotos der Messorte angegeben. Die Fotos auf der rechten Seite geben einen senkrecht zur Hautoberfläche gelegten Schnitt durch ein in Paraffin eingebettetes malignes Melanom von 7,7 mm Dicke wider. Der helle Fleck darin zeigt den jeweiligen Messort bei oben 0 mm, in der Mitte 3 mm und unten 6 mm Messtiefe. Die mit den oben angegebenen Parametern ermittelten Fluoreszenzspektren sind auf der linken Seite dargestellt. Die Messeinrichtung ist so eingestellt, dass das Messareal jeweils einen Durchmesser von 70 µm aufweist. Die Fluoreszenzspektren oben und unten, unmittelbar an der Hautoberfläche und direkt am unteren Ende des malignen Melanoms, zeigen das Spektrum gesunden Hautgewebes mit der charakteristischen Ausprägung des Eumelanins bei 475 nm. Das Fluoreszenzspektrum in der Mitte, im Zentrum des malignen Melanoms, zeigt sowohl die relative Ausprägung des für Pheomelanin, d.h. für das maligne Melanom, charakteristischen Anteils bei 575 nm und die Lücke bei 525 nm als Nachweis der Unterdrückung der Flavin-Fluoreszenz, als auch die Abnahme der Fluoreszenz-Gesamtintensität im malignen Melanom. **Figur 1B** zeigt 2 Beispiele für Fluoreszenzspektren eines malignen Melanoms mit Zweiphotonen-Anregung und Anregungspulsen bei auf 880 nm verschobener Wellenlänge und 2 ns Pulslänge mit Fotos der Messorte bei oben 0 mm und unten 2 mm Messtiefe und einem Durchmesser von 70 µm. Die ermittelten Fluoreszenzspektren sind wieder auf der linken Seite dargestellt. Die Messergebnisse demonstrieren, dass das maligne Melanom auch noch bei der Anregung mit einer nahe bei 900 nm liegenden Wellenlänge sicher identifiziert werden kann, mit dem entscheidenden Vorteil einer insbesondere für in-vivo-Anwendungen risikofreien Messung, da selbst 3-Photonenabsorption, die unter den vorliegenden Meßbedingungen ohnehin eine zu vernachlässigende Wahrscheinlichkeit hat, nicht in den UV-C Bereich führt. Infolge der bedingt durch die Verlängerung der Anregungswellenlänge energieärmeren Anregung ist das Autofluoreszenzspektrum insgesamt etwas bathochrom verschoben, die charakteristischen Maxima von 475 nm nach 525 nm, bzw. von 575 nm nach 600 nm. Das Fluoreszenzspektrum oben zeigt wieder das Spektrum gesunden Hautgewebes mit der bathochrom verschobenen Ausprägung des *Eumelanins* bei 525 nm. Das Fluoreszenzspektrum unten zeigt wieder sowohl das Spektrum für das maligne Melanom mit der bathochrom verschobenen Ausprägung des *Pheomelanins* bei 600 nm als auch erneut die Abnahme der Fluoreszenz-Gesamtintensität im malignen Melanom.

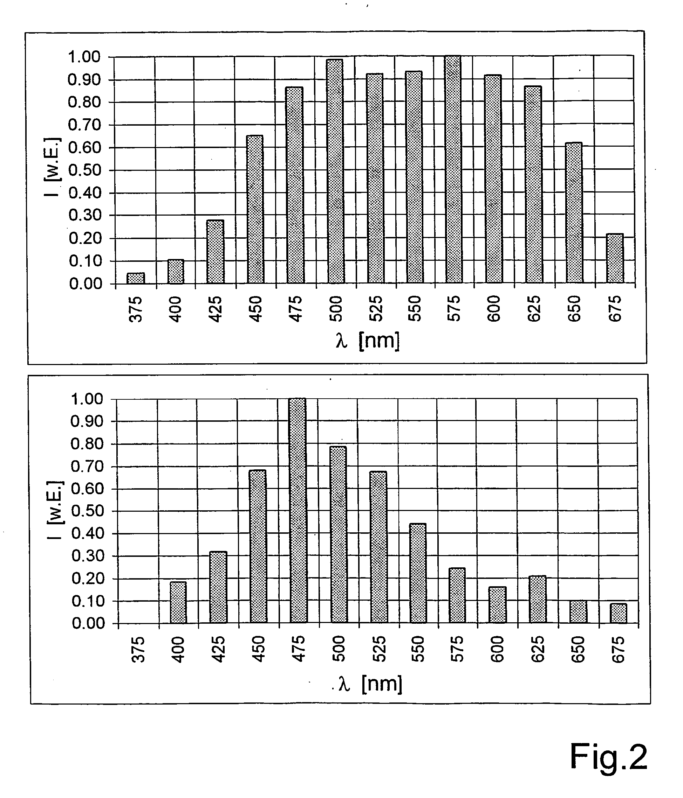

In **Figur 2** werden nochmals die Fluoreszenzspektren von mit dem malignen Melanom befallenem und gesundem Hautgewebe direkt gegenübergestellt. Die Anregungsparameter sind: Zweiphotonen-Anregung bei 810 nm Wellenlänge und 0,7 ns Pulsdauer mit einem Durchmesser des Messorts von 70 µm. Die Proben stammen von verschiedenen Messvorgängen und damit sind die beiden Fluoreszenzspektren nur qualitativ, nicht aber quantitativ vergleichbar. Das untere Fluoreszenzspektrum stammt von einer Probe gesunder Haut und zeigt die charakteristische spektrale Verteilung der Fluoreszenz mit einem deutlichen Schwerpunkt bei 475 nm für *Eumelanin.* Das obere Fluoreszenzspektrum stammt von einer Probe mit malignem Melanom und zeigt eine deutliche Zunahme der Fluoreszenz bei 575 nm für *Pheomelanin,* dem Indikator für das maligne Melanom sowie der charakteristischen Lücke bei 525 nm für die Flavin-Fluoreszenz-Unterdrückung. Die in solchen Fällen auftretende Reduzierung der Gesamtfluoreszenz ist in diesem Beispiel nicht sichtbar, da die beiden Fluoreszenzspektren wegen der Abstammung von verschiedenen Messvorgängen nicht zueinander normierbar sind. Z.B. können schon die Dicken der zu überwindenden Paraffin-Schichten über den Hautproben unterschiedlich sein. Unabhängig davon sind die resultierenden Fluoreszenzspektren der beiden Proben qualitativ eindeutig befallenem und gesundem Hautgewebe zuordenbar und zeigen, dass auch bei Detektionen mit ganz unterschiedlichen Bedingungen bei Einhaltung gleicher Pulsparameter der Fluoreszenzanregung eine eindeutige Aussage über den Zustand der Probe am Messort allein auf Grund des spektralen Effekts getroffen werden kann.

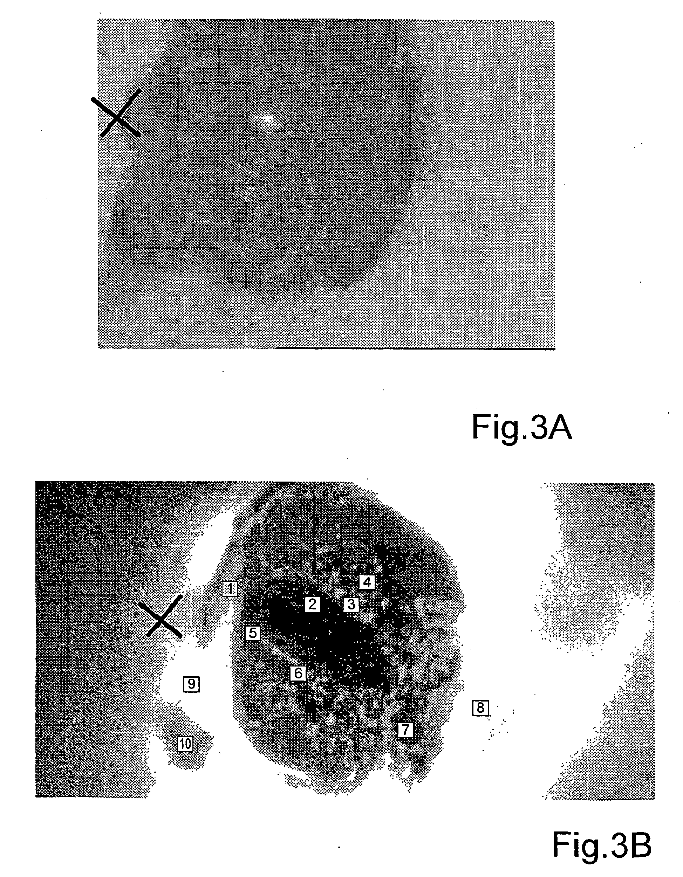

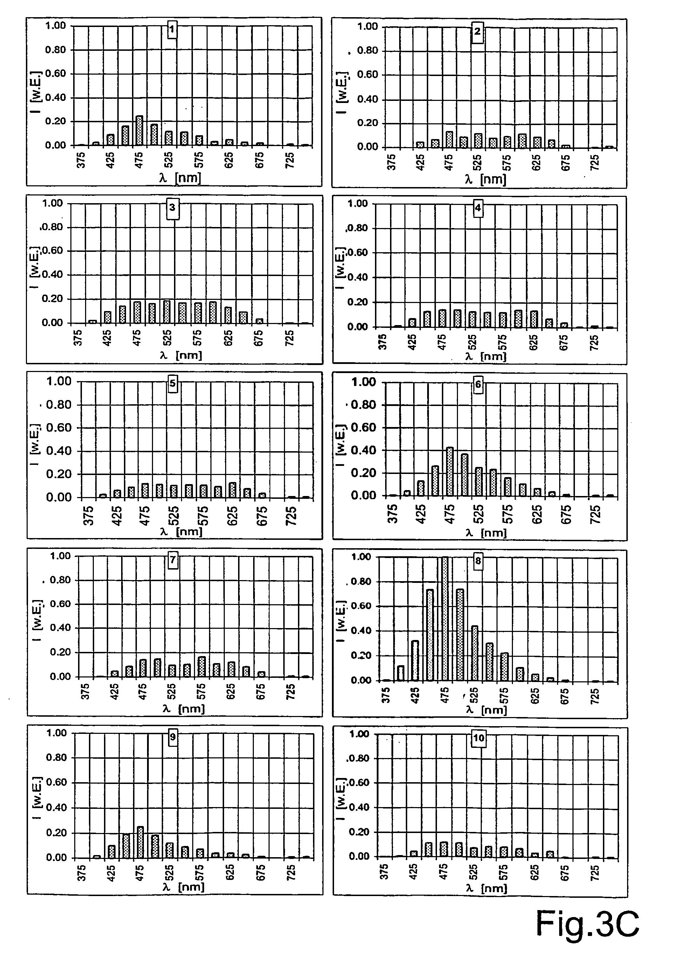

**Figur 3A** zeigt ein Foto mit normalem Licht eines in Paraffin eingebetteten Hautgewebe-Areals mit einem malignen Melanom wie in Figur 1. Mit dem Kreuz ist ein markanter Punkt an der Hautoberfläche der Probe gekennzeichnet. Zum Größenvergleich beträgt die Distanz vom Kreuz bis zum Lichtspot 3mm. **Figur 3B** zeigt nun zunächst zur Abschätzung von Verdachtsarealen eine Grauwert-Aufnahme als Übersicht desselben Meßojektes wie in Figur 3A, hier im Lichte der mit 337 nm angeregten Fluoreszenz. Zum Vergleich mit Figur 3A ist das Kreuz an identischem Ort eingetragen. Markiert sind darin 10 Messorte, deren Fluoreszenz-Spektren nachfolgend auch in **Figur 3C** dargestellt sind. Das dunkle Verdachtsareal für ein malignes Melanom ist insbesondere um den Messort 2 herum deutlich sichtbar. Die Fluoreszenzspektren an den 10 Messorten wurden mit Impulsen mit 810 nm Wellenlänge und 0,7 ns Pulsdauer angeregt. Das fluoreszierende Areal hat jeweils einen Durchmesser von 70 µm. Die Ordinate der Spektren ist normiert auf das Maximum der gesamten Meßreihe, d.h. neben der spektralen Variation ist auch die Intensitätsvariation über die Messorte sichtbar. Der außerhalb des durch die Übersichtsaufnahme insbesondere am Messpunkt 2 feststellbaren Verdachtsareals liegende Messort 8 erweist sich auch bei der spektralen Analyse als besonders unverdächtig und dient mit seiner ungestörten Fluoreszenz gesunden Hautgewebes als Normierungsreferenz für alle übrigen neun Fluoreszenzspektren in dieser Figur. Am Messort 9 wird zwar noch die ungestörte spektrale Verteilung gesunden Hautgewebes mit dem praktisch kontinuierlichen Verlauf des Messwertäbnahme zwischen 475 und 675 nm detektiert, allerdings mit einer deutlichen Abschwächung der Gesamtfluoreszenz, womit schon ein Grundverdacht auf eine Störung, jedoch noch nicht auf ein mit malignem Melanom im Anfangsstadium befallenes Hautareal besteht. Die Messorte 1 und 6 zeigen eine kleine, aber gut erkennbare Abweichung von diesem kontinuierlichen Verlauf jeweils bei 550 nm und erregen damit schon einen erhöhten Verdacht auf einen Befall des zugehörigen Hautareals mit einem malignem Melanom im Anfangsstadium. Die Fluoreszenzspektren an den übrigen Messorten 2, 3, 4, 5, 7 und 10 zeigen ausgeprägt die für den Befall mit dem malignen Melanom typischen Ausprägungen der spektralen Verteilung mit Maxima um 575 nm herum, der charakteristischen Lücke um 525 nm herum für die Flavin-Fluoreszenz-Unterdrückung und der deutlichen Abschwächung der Gesamtfluoreszenz.

**Figur 4A** zeigt eine Messanordnung **MG** für die Aufnahme von Bildern vom Messobjekten im Licht ihrer mit Einphotonen-Anregung erzeugten Fluoreszenz. Der Anregungslaser **AL** sendet Pulse **LP** von Laserlicht mit einer Wellenlänge von z.B. 337 nm bei einer Pulslänge von 2,5 ns aus. Die Pulse **LP** werden unfokussiert durch ein Bündel von Lichtleitfasern **BL** auf ein Messareal **MA** mit einem Durchmesser von beispielsweise 1 cm geleitet. Das durch die Pulse **LP** hervorgerufene Fluoreszenzlicht wird durch einen Filter auf eine Aufnahmekamera **AK** geleitet dort in ein den Intensitäten der aufgenommenen Wellenlängen proportionales Grauwert- oder Falschfarbenbild umgesetzt. Das Bild wird auf einer Auswerteinheit **AE** dargestellt, gespeichert und für weitere Verarbeitungen bereitgehalten. Mit den so aufgenommenen Bildern können z.B. größere Hautbereiche für eine erste Abschätzung auf Verdachtsareale für das maligne Melanom gescannt werden. Es handelt sich nach den in der Beschreibung und in den Figuren 1 bis 3 dargestellten eindeutigen Bewertungskritereien um ein rein techisches Messverfahren, das von einem angelernten Techniker oder zukünftig sogar von einem entsprechend gestalteten Programm bedient werden kann und liefert vollkommen objektive Ergebnisse. **Figur 4B** zeigt eine Messanordnung **MG** für die Aufnahme und Verarbeitung der Fluoreszenzspektren mit Zweiphotonen-Anregung. Der im Wellenlängenbereich zwischen 600 und 1000 nm durchstimmbare Anregungslaser **AL** sendet Pulse **LP** von Laserlicht mit einer Wellenlänge von z.B. um 850 nm herum bei einer Pulslänge im Bereich von 0,7 bis 2,5 ns aus. Die Pulse **LP** werden von einem dielektrischen Spiegel **DS**, der für bestimmte Wellenlängen wie ein Spiegel wirkt und für andere durchlässig ist, abgelenkt und von einem Linsensystem **LS** auf den Messspot **MS** mit einem Durchmesser von beispielsweise 70 µm fokussiert. Im Messspot **MS** wird so der Melaninanteil am Fluorophorgemisch selektiv und ortsaufgelöst zur Fluoreszenz angeregt. Das abgestrahlte Fluoreszenzspektrum im Wellenlängenbereich zwischen 400 und 700 nm wird wieder durch das Linsensystem **LS** auf den dielektrischen Spiegel **DS** zurückgeleitet, durch diesen durchgelassen und von einem weiteren Linsensystem **WL** mit Filter auf den Eingang eines Bündels von Lichtleitfasern **BL** fokussiert. Das Bündel **BL** leitet das Fluoreszenzlicht in ein Spektrometer **SM**, in dem das aufgenommene Fluoreszenzspektrum zerlegt und die Intensität der Wellenlängen in Schritten von beispielsweise 25 nm detektiert wird. Ein Sekundärelektronen-Vervielfacher **SV** verstärkt das Messergebnis und leitet es abschließend in eine Auswerteeinheit **AE**, die es darstellt, speichert und für weitere Verarbeitungen bereithält. Mit den so aufgenommenen Fluoreszenzspektren können z.B. Messspots aus den mit der in Figur 4A dargestellten Messanordnung ermittelten Verdachtsarealen konkret und objektiv auf das Vorhandensein von mit dem malignen Melanom befallenenen Hautregionen getestet werden. Es handelt sich auch hier nach den in der Beschreibung und den Figuren 1 bis 3 gezeigten eindeutigen Bewertungskritereien um ein rein technisches Messverfahren, das von einem angelernten Techniker oder einem Programm bedient werden kann. Es liefert vollkommen objektive, von Recheneinheiten bewertbare Ergebnisse. Die beschriebenen Messverfahren sind in ihrer Gesamtheit also rein technischer Natur und unterliegen in erster Linie klaren und objektiven Beurteilungsmaßstäben angelernten Bedienungspersonals.

## Patentansprüche

1. Ortsaufgelöstes Messverfahren für die Detektion von Melanin in Fluorophorgemischen in einer Festkörperprobe mit den Verfahrensschritten:
1. ausschließliche Fluoreszenzanregung des im Fluorophorgemisch vorhandenen Melanins durch Photonen-Absorption
1.1 mit zumindest einem Puls einer Laserlichtquelle,
1.2 mit einer Pulslänge im Bereich zwischen 0,5 und 5 ns,
1.3 mit einer Anregungswellenlänge im Bereich zwischen 300 und 1000 nm,
1.4 mit einer Photonenflussdichte im Bereich zwischen 10²⁶ und 10²⁸ Photonen pro cm² und s
2. Erfassung des im Fluophorgernisch vorhandenen Melanins aus dessen ausgesendeter spektraler Fluoreszenzantwort durch Auswertung der Anzahlen der ausgesendeten Photonen
2.1 bei Fluoreszenzwellenlängen im Bereich zwischen 400 und 700 nm.

2. Ortsaufgelöstes Messverfahren nach Anspruch 1,
**dadurch gekennzeichnet, dass**
die Pulslänge 2 ns und die Photonenflussdichte 10²⁷ Photonen pro cm² und s beträgt.

3. Ortsaufgelöstes Messverfahren nach Anspruch 1 oder 2,
dadurch gekenntzeichnet, dass
eine Mittelung über eine Akkumulationsanzahl im Bereich zwischen 2 und 100 einzelnen Pulsen der Laserlichtquelle durchgeführt wird.

4. Ortsaufgelöstes Messverfahren nach einem der Ansprüche 1 bis 3,
**dadurch gekennzeichnet, dass**
die spektrale Fluoreszenzantwort in Wellenlängenschritten von 25 nm erfasst wird.

5. Ortsaufgelöstes Messverfahren nach einem der Ansprüche 1 bis 3,
**dadurch gekennzeichnet, dass**
die Probe eine Größe in einem Bereich von 1 cm² aufweist.

6. Ortsaufgelöstes Messverfahren nach einem der Ansprüche 1 bis 5,
**dadurch gekennzeichnet, dass**
1. die Photonen-Absorption durch Anregungswellenlängen im Bereich zwischen 300 und 350 nm ausschließlich als Ein-Photonen-Absorption erfolgt und
2. die Erfassung der Fluoreszenzantwort global über die Festkörperprobe durch eine bildliche Darstellung erfolgt.

7. Ortsaufgelöstes Messverfahren nach Anspruch 6,
**dadurch gekennzeichnet, dass**
die Anregungswellenlänge 337 nm beträgt.

8. Ortsaufgelöstes Messverfahren nach Anspruch 6 oder 7,
**dadurch gekennzeichnet, dass**
die bildliche Darstellung durch direkte optische Fotografie der im sichtbaren Wellenlängenbereich abgestrahlten Fluoreszenz erfolgt.

9. Ortsaufgelöstes Messverfahren nach einem der Ansprüche 1 bis 5,
**dadurch gekennzeichnet, dass**
1. die Photonen-Absorption durch Anregungswellenlängen im Bereich zwischen 600 und 1000 nm ausschließlich als stufenweise Zwei-Photonen-Absorption erfolgt und
2. die Erfassung der Fluoreszenzantwort lokal und selektiv bei der für die Melaninsorte *Eumelanin* charakteristischen Fluoreszenzwellenlänge von 475 nm und bei der für die Melaninsorte *Pheomelanin* charakteristischen Fluoreszenzwellenlänge von 575 nm erfolgt.

10. Ortsaufgelöstes Messverfahren nach Anspruch 9,
**dadurch gekennzeichnet, dass**
die Anregungswellenlänge im Bereich zwischen 800 und 900 nm liegt.

11. Ortsaufgelöstes Messverfahren nach Anspruch 9 oder 10,
**dadurch gekennzeichnet, dass**
die lokale Ortsauflösung bei Messspots mit Durchmessern im Bereich zwischen 40 und 100 µm liegt.

12. Ortsaufgelöstes Messverfahren nach je einem der Ansprüche 6 bis 8 und 9 bis 11,
**gekennzeichnet durch** die Verfahrensschritte :
1. Durchführung der Ein-Photonen-Absorption mit einer bildlichen Erfassung der Fluoreszenzantwort global über die Festkörperprobe,
2. Auswahl spezieller Areale der Probe **durch** Feststellung von Fluoreszenzauslöschung in der Fluoreszenzantwort
3. Durchführung der stufenweisen Zwei-Photonen-Absorption mit einer selektiven Erfassung der Fluoreszenzantwort lokal in den ausgewählten Arealen der Festkörperprobe und
4. Ermittlung des auftretenden Anteils der Melaninsorte *Pheomelanin* **durch** Festellung der zugehörigen Anzahl der ausgestrahlten Photonen

13. Ortsaufgelöstes Messverfahren nach Anspruch 12,
**dadurch gekennzeichnet, dass**
der Grad der Fluoreszenzauslöschung durch proportional dazu auftretende Farb- oder Grauabstufungen ermittelt wird, wobei die dunkelsten Areale ausgewählt werden.

14. Ortsaufgelöstes Messverfahren nach Anspruch 12 oder 13,
**dadurch gekennzeichnet, dass**
das Verhältnis der auftretenden Anteile der Melaninsorte *Pheomelanin* und der Melaninsorte *Eumelanin* registriert wird.

15. Ortsaufgelöstes Messverfahren nach einem der Ansprüche 1 bis 14,
**dadurch gekennzeichnet, dass**
als Festkörperproben Gewebeteile der menschlichen Haut, des menschlichen Augenhintergrunds oder der menschlichen Haare verwendet werden.

16. Ortsaufgelöstes Messverfahren nach Anspruch 15,
**dadurch gekennzeichnet, dass**
das Verfahren ex-vivo an frisch biopsierten Festkörperproben durchgeführt wird und die Festkörperproben in Paraffin stabilisiert und in Formalin fixiert sind.

17. Ortsaufgelöstes Messverfahren nach Anspruch 15,
**dadurch gekennzeichnet, dass**
das Verfahren in-vivo durchgeführt wird und die Festkörperproben in ihrer natürlichen Umgebung fixiert sind.

## Claims

1. A spatially resolved measurement method for the detection of melanin in fluorophore mixtures in a solid sample comprising the method steps:
1. exclusive fluorescence excitation of the melanin present in the fluorophore mixture by means of photon absorption 1.1 with at least one pulse of a laser light source,
1.2 with at a pulse length in the range between 0.5 to 5 ns,
1.3 with an excitation wavelength in the range between 300 and 1000 nm,
1.4 with a photon flux density in the range between 10²⁶ and 10²⁸ photons per cm² and s
2. detection of the melanin present in the fluorophore mixture on the basis of its emitted spectral fluorescence response by evaluating the numbers of emitted photons
2.1 at fluorescence wavelengths in the range between 400 nm and 700 nm.

2. The spatially resolved measurement method according to claim 1,
**characterised in that**
the pulse length amounts to 2 ns and the photon flux density amounts to 10²⁷ photons per cm² and s.

3. The spatially resolved measurement method according to claim 1 or 2,
**characterised in that**
an averaging over an accumulation number in the range between 2 and 100 individual pulses of the laser light source is carried out.

4. The spatially resolved measurement method according to any one of claims 1 to 3,
**characterised in that**
the spectral fluorescence response is detected in wavelength increments of 25 nm.

5. The spatially resolved measurement method according to any one of claims 1 to 3,
**characterised in that**
the sample has a size in the region of 1 cm².

6. The spatially resolved measurement method according to any one of claims 1 to 5,
**characterised in that**
1. the photon absorption takes place through excitation wavelengths in the range between 300 and 350 nm exclusively as a one-photon absorption and
2. the fluorescence response is ascertained over the entire solid sample in the form of a visual depiction.

7. The spatially resolved measurement method according to claim 6,
**characterised in that**
the excitation wavelength is 337 nm.

8. The spatially resolved measurement method according to claim 6 or 7,
**characterised in that**
the visual depiction is made by means of direct optical photography of the fluorescence emitted in the visible wavelength region.

9. The spatially resolved measurement method according to any one of claims 1 to 5,
**characterised in that**
1. the photon absorption is effected by excitation wavelengths in the range between 600 and 1000 nm exclusively as stepwise two-photon absorption and
2. the fluorescence response is ascertained locally and selectively at the fluorescence wavelength of 475 nm characteristic of the melanin type *Eumelanin* and at the fluorescence wavelength of 575 nm characteristic of the melanin type *Pheomelanin.*

10. The spatially resolved measurement method according to claim 9,
**characterised in that**
the excitation wavelength lies in the range between 800 and 900 nm.

11. The spatially resolved measurement method according to claim 9 or 10,
**characterised in that**
the local spatial resolution lies at measuring spots with diameters in the range between 40 and 100 µm.

12. The spatially resolved measurement method according to any one of claims 6 to 8 and 9 to 11,
**characterised by** the method steps:
1. carrying out the one-photon absorption by imaging the fluorescence response over the entire solid sample,
2. selecting specific areas of the sample by ascertaining the fluorescence extinction in the fluorescence response,
3. carrying out the stepwise two-photon absorption with a selective detection of the fluorescence response locally in the selected areas of the solid sample, and
4. determining the occurring fraction of the melanin type *Pheomelanin* by ascertaining the appertaining number of emitted photons.

13. The spatially resolved measurement method according to claim 12,
**characterised in that**
the degree of the fluorescence extinction is ascertained on the basis of colour or greyscale gradations occurring proportionally thereto, wherein the darkest areas are selected.

14. The spatially resolved measurement method according to claim 12 or 13,
**characterised in that**
the ratio of the occurring fractions of the melanin type *Pheomelanin* and the melanin type *Eumelanin* is recorded.

15. The spatially resolved measurement method according to any one of claims 1 to 14,
**characterised in that**
tissue parts of human skin, the fundus of the human eye or human hairs are used as solid samples.

16. The spatially resolved measurement method according to claim 15,
**characterised in that**
the method is carried out ex-vivo on freshly biopsied solid samples and the solid samples are stabilised in paraffin and fixed in formalin.

17. The spatially resolved measurement method according to claim 15,
**characterised in that**
the method is carried out in-vivo and that the solid samples are fixed in their natural environment.

## Revendications

1. Procédé de mesure à résolution locale pour la détection de mélanine dans des mélanges de fluorophores dans un échantillon de corps solide comportant les étapes de procédé:
1. excitation de fluorescence exclusive de la mélanine présente dans le mélange de fluorophores par absorption de photons 1.1 avec au moins une impulsion d'une source de lumière laser,
1.2 avec une longueur d'onde dans la plage comprise entre 0,5 et 5 ns,
1.3 avec une longueur d'onde d'excitation comprise dans la plage entre 300 et 1000 nm,
1.4 avec une densité de flux de photons comprise dans la plage entre 10²⁶ et 10²⁸ photons par cm² et s
2. détection de la mélanine présente dans le mélange de fluorophores d'après sa réponse de fluorescence spectrale émise en évaluant les nombres de photons émis
2.1 à des longueurs d'ondes de fluorescence comprise dans la plage entre 400 et 700 nm.

2. Procédé de mesure à résolution locale selon la revendication 1,
**caractérisé en ce que**
la longueur d'impulsion s'élève à 2 ns et la densité de flux de photons s'élève à 10²⁷ photons par cm² et s.

3. Procédé de mesure à résolution locale selon les revendications 1 ou 2,
**caractérisé en ce que**
un calcul de la moyenne est effectué au moyen d'un nombre d'accumulation compris dans la plage entre 2 et 100 impulsions individuelles de la source de lumière laser.

4. Procédé de mesure à résolution locale selon une des revendications 1 à 3,
**caractérisé en ce que**
la réponse de fluorescence spectrale est détectée à des longueurs d'ondes de 25 nm.

5. Procédé de mesure à résolution locale selon une des revendications 1 à 3, **caractérisé en ce que**
l'échantillon présente une taille dans une plage de 1 cm².

6. Procédé de mesure à résolution locale selon une des revendications 1 à 5,
**caractérisé en ce que**
1. l'absorption de photons par des longueurs d'ondes d'excitation a lieu dans la plage comprise entre 300 et 350 nm exclusivement comme absorption à un photon et
2. la détection de la réponse de fluorescence a lieu globalement sur l'échantillon de corps solide par une représentation schématique.

7. Procédé de mesure à résolution locale selon la revendication 6,
**caractérisé en ce que**
la longueur d'onde d'excitation s'élève à 337 nm.

8. Procédé de mesure à résolution locale selon les revendications 6 ou 7,
**caractérisé en ce que**
la représentation schématique a lieu par photographie optique directe de la fluorescence émise dans la plage de longueur d'onde visible.

9. Procédé de mesure à résolution locale selon une des revendications 1 à 5,
**caractérisé en ce que**
1. l'absorption de photons par des longueurs d'ondes d'excitation dans la plage comprise entre 600 et 1000 nm a lieu exclusivement comme une absorption à deux photons graduelle et
2. la détection de la réponse de fluorescence a lieu localement et sélectivement sur la longueur d'onde de fluorescence de 475 nm caractéristique du type de mélanine *Eumélanine* et sur la longueur d'onde de fluorescence 575 nm caractéristique du type de mélanine *Phéomélanine.*

10. Procédé de mesure à résolution locale selon la revendication 9,
**caractérisé en ce que**
la longueur d'onde d'excitation est située dans la plage comprise entre 800 et 900 nm.

11. Procédé de mesure à résolution locale selon les revendications 9 ou 10,
**caractérisé en ce que**
la résolution locale est située à des points de mesure avec des diamètres dans la plage comprise entre 40 et 100 µm.

12. Procédé de mesure à résolution locale selon respectivement une des revendications 6 à 8 et 9 à 11,
**caractérisé par les étapes de procédé:**
1. mise en oeuvre de l'absorption à un photon avec une détection schématique de la réponse de fluorescence globalement sur l'échantillon de corps solide,
2. sélection d'aires spéciales de l'échantillon en constant l'effacement de la fluorescence dans la réponse de fluorescence,
3. mise en oeuvre de l'absorption à deux photons graduelle avec une détection sélective de la réponse de fluorescence localement dans les aires sélectionnées de l'échantillon de corps solide et
4. détermination de la fraction apparue du type de mélanine *Phéomélanine* en constatant le nombre correspondant de photons émis.

13. Procédé de mesure à résolution locale selon la revendication 12,
**caractérisé en ce que**
le degré d'effacement de fluorescence est déterminé par des échelles de gris ou de couleur apparaissant proportionnellement, dans lequel les aires les plus sombres sont sélectionnées.

14. Procédé de mesure à résolution locale selon les revendications 12 ou 13,
**caractérisé en ce que**
le rapport des fractions apparues du type de mélanine *Phéomélanine* et du type de mélanine *Eumélanine* est enregistré.

15. Procédé de mesure à résolution locale selon une des revendications 1 à 14,
**caractérisé en ce que**
comme échantillons de corps solide, on utilise des morceaux de tissu de peau humaine, du fond d'oeil humain ou de cheveux humains.

16. Procédé de mesure à résolution locale selon la revendication 15,
**caractérisé en ce que**
le procédé est mis en oeuvre ex-vivo sur des échantillons de corps solide fraîchement biopsiés et les échantillons de corps solide sont stabilisés dans la paraffine et fixés dans la formaline.

17. Procédé de mesure à résolution locale selon la revendication 15,
**caractérisé en ce que**
le procédé est mis en oeuvre in vivo et les échantillons de corps solide sont fixés dans leur environnement naturel.
